(19) Europäisches Patentamt
European Patent Office
Office européen des brevets

(11) EP 4 256 960 A1

(12) **EUROPEAN PATENT APPLICATION**
published in accordance with Art. 153(4) EPC

(43) Date of publication:
**11.10.2023 Bulletin 2023/41**

(21) Application number: **22842453.7**

(22) Date of filing: **13.07.2022**

(51) International Patent Classification (IPC):
***A01N 33/12*** *(2006.01)*

(52) Cooperative Patent Classification (CPC):
**A01N 33/12; A01P 1/00; C12Q 1/18;** Y02A 50/30

(86) International application number:
**PCT/KR2022/010196**

(87) International publication number:
**WO 2023/287191 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2021 KR 20210093569**

(71) Applicant: **Lg Chem, Ltd.**
**Seoul 07336 (KR)**

(72) Inventors:
- **KANG, Yeonjoo**
  **Daejeon 34122 (KR)**
- **LEE, Ji Seok**
  **Daejeon 34122 (KR)**
- **KANG, Soonhee**
  **Daejeon 34122 (KR)**
- **OH, Sung Joon**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **ANTIBACTERIAL COMPOSITION**

(57) An antibacterial composition according to an exemplary embodiment of the present invention comprises one or more compounds comprising a quaternary ammonium structure having an acrylate group or a methacrylate group, in which a critical micelle concentration of the compound comprising the quaternary ammonium structure is 0.1 wt% to 3 wt%.

[Figure 4]

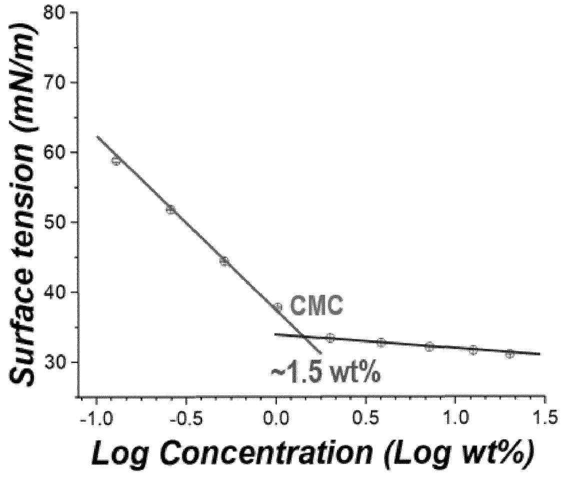




Processed by Luminess, 75001 PARIS (FR)

## Description

[Technical Field]

The present invention claims priority to and the benefit of Korean Patent Application No. 10-2021-0093569 filed in the Korean Intellectual Property Office on July 16, 2021, the entire contents of which are incorporated herein by reference.

The present invention relates to an antibacterial composition.

[Background Art]

Recently, various products such as daily supplies or hygiene products are required to have antibacterial properties.

The degree of antibacterial properties required and the material requirements for imparting antibacterial properties differ depending on the material of the product requiring antibacterial properties and the state of final use. For example, the properties of the material for imparting antibacterial properties and the degree of antibacterial properties vary depending on the amount of antibacterial material applied to a product and the materials used together.

Therefore, there is a need for developing an antibacterial material suitable for application to each of various products.

[Detailed Description of the Invention]

[Technical Problem]

The present invention has been made in an effort to provide an antibacterial composition comprising an antibacterial material which has hydrophilicity and hydrophobicity, and thus, is advantageous for imparting antibacterial properties, and is capable of not only imparting antibacterial properties but also solving safety problems due to the leakage of antibacterial materials, by forming a copolymer with an acryl-based resin and the like.

The present invention has also been made in an effort to provide an antibacterial composition capable of adjusting the surface tension, that is, increasing the solubility in solvents by adjusting the number of carbon atoms comprised in an antibacterial monomer.

[Technical Solution]

An exemplary embodiment of the present invention provides an antibacterial composition comprising:

one or more compounds comprising a quaternary ammonium structure having an acrylate group or a methacrylate group,
in which a critical micelle concentration of the compound comprising the quaternary ammonium structure is 0.1 wt% to 3 wt%.

Further, another exemplary embodiment of the present invention provides a product comprising the antibacterial composition or prepared therefrom.

[Advantageous Effects]

The antibacterial composition according to some exemplary embodiments of the present invention is an antibacterial composition comprising an antibacterial material which has hydrophilicity and hydrophobicity, and thus, is advantageous for imparting antibacterial properties, and is capable of not only imparting antibacterial properties but also solving safety problems due to the leakage of antibacterial materials, by forming a copolymer with an acryl-based resin, has antibacterial properties and acute oral toxicity dose controlled within a characteristic range, and is useful as a material capable of safely imparting excellent antibacterial properties.

The antibacterial composition according to some exemplary embodiments of the present invention can exhibit antibacterial properties within a short period of time.

Since the antibacterial composition according to some exemplary embodiments of the present invention has little change in antibacterial strength depending on the amount of antibacterial material used, antibacterial properties can be exhibited within an expected range even when the unevenness of concentration occurs unintentionally during application to a product.

The antibacterial composition according to some exemplary embodiments of the present invention has an advantage of high solubility in solvents.

The antibacterial composition according to some exemplary embodiments of the present invention has low toxicity properties.

[Brief Description of Drawings]

FIG. 1 is a view illustrating the $^1$H-NMR spectrum ($(CD_3)_2SO$) of synthesized Monomer 1 as an exemplary embodiment of the present invention.

FIG. 2 is a view illustrating the $^1$H-NMR spectrum ($(CD_3)_2SO$) of synthesized Monomer 2 as an exemplary embodiment of the present invention.

FIG. 3 is a view illustrating the $^1$H-NMR spectrum ($(CD_3)_2SO$) of synthesized Monomer 3 as an exemplary embodiment of the present invention.

FIG. 4 is a view illustrating the surface tension of Monomer 2 at each concentration as an exemplary embodiment of the present invention.

FIG. 5 is a view illustrating the surface tension of Monomer 3 at each concentration as an exemplary embodiment of the present invention.

FIG. 6 is a view illustrating the surface tension of Comparative Monomer 2 at each concentration.

[Best Mode]

An exemplary embodiment of the present invention provides an antibacterial composition comprising one or more compounds comprising a quaternary ammonium structure having an acrylate group or a methacrylate group, in which a critical micelle concentration of the compound comprising the quaternary ammonium structure is 0.1 wt% to 3 wt%.

According to another exemplary embodiment of the present invention, the antibacterial composition has an antibacterial strength A of 90% or more which are measured by the following Method 1 for at least one strain of Gram-positive bacteria and Gram-negative bacteria and an acute oral toxicity dose LD50 of 300 mg/Kg or more.

[Method 1]

After 25 ml of a broth-type medium (Nutrient broth, BD DIFCO., 8 g/L) inoculated with 3,000 CFU/ml bacteria is put into a 50 mL conical tube, 0.015 g of the antibacterial composition is added thereto and suspended (vortexing), a sufficiently mixed solution is incubated for 16 hours in a shaking water bath maintained at 35°C.

After the incubated solution was diluted to 1/5 using a 1X PBS buffer solution, absorbance ($\lambda$ = 600 nm) is measured using a UV/Vis spectrophotometer, and the antibacterial strength, which is a bacteriostatic reduction rate, is calculated by the following equation by comparing the measured absorbance with a solution incubated without the addition of the antibacterial composition.

$$\text{Antibacterial strength (\%)} = \left(1 - A_{sample}/A_{Reference}\right) \times 100$$

$$A_{sample} = \text{Absorbance of medium solution incubated by adding antibacterial composition}$$

$$A_{Reference} = \text{Absorbance of medium solution incubated without addition of antibacterial composition}$$

A compound comprising a quaternary ammonium structure having an acrylate group or methacrylate group

comprised in the antibacterial composition may physicochemically destruct and kill a cell surface layer structure by hydrophobic interaction, when cations of the ammonium molecule of the quaternary ammonium-based compound are electrostatically adsorbed onto anionic sites of the cell surface of bacteria or microorganisms and a substituent bound to the quaternary ammonium structure is hydrophobic. Further, an antibacterial function may be introduced through copolymerization with an acryl-based resin and the like by the acrylate group or methacrylate group, and in this case, safety may be improved by preventing the antibacterial material from being leaked. According to the exemplary embodiments of the present invention, high antibacterial properties may be safely imparted by comprising compounds having the quaternary ammonium structure as described above and simultaneously limiting the antibacterial strength and acute oral toxicity dose of the antibacterial composition to within a specific range. Furthermore, according to the exemplary embodiments of the present invention, the surface tension may be adjusted by adjusting the number of carbon atoms of the compound having the quaternary ammonium structure as described above. Specifically, there is an advantage in that the critical micelle concentration can be obtained within a target range. The target critical micelle concentration range is the same as 0.1 wt% to 3 wt% described above.

The antibacterial strength mentioned in the present specification may be measured for at least one strain of *Proteus mirabilis, E.coli, S.aureus, E.Cloaceae* and *E.faecalis.* Preferably, the antibacterial strength mentioned in the present specification may be measured for each of *Proteus mirabilis, Ecoli, S.aureus, E.Cloaceae* and *E.faecalis.* The antibacterial composition according to exemplary embodiments of the present invention may have a certain level or more of antibacterial strength against Gram-positive bacteria and Gram-negative bacteria. ATCC29906 and CCUG4637 may be used as *Proteus mirabilis,* ATCC13047, ATCC13048, and CCUG71839 may be used as *E.Cloaceae,* and ATCC29212 and CCUG9997 may be used as *E.faecalis.*

According to another exemplary embodiment of the present invention, the antibacterial strength A of the antibacterial composition is expressed within 1 hour. This enables a desired antibacterial strength to be exhibited immediately after application of the antibacterial composition to an intended use or product.

According to still another exemplary embodiment of the present invention, the ratio (CB) of the antibacterial strength C measured by the following Method 3 to the antibacterial strength B measured by the following Method 2 of the antibacterial composition is 1 or more and less than 2:

[Method 2]

This method is the same as Method 1, except that 0.005 g of the antibacterial composition is added instead of 0.015 g of the antibacterial composition.

[Method 3]

This method is the same as Method 1, except that 0.02 g of the antibacterial composition is added instead of 0.015 g of the antibacterial composition.

When the antibacterial composition is applied to the intended use or product, regions of unintentionally high concentration and low concentration of the antibacterial material may occur, and a region of low concentration may not exhibit the desired antibacterial properties. However, according to the exemplary embodiments, since there is little change in antibacterial strength depending on the amount of antibacterial material used, antibacterial properties can be exhibited within an expected range even when the unevenness of concentration occurs unintentionally during application to an intended use or product.

According to yet another exemplary embodiment of the present invention, the antibacterial strength A is 99% or more, preferably 99.3% or more, more preferably 99.5% or more, even more preferably 99.7% or more, and still even more preferably 99.9% or more.

According to yet another exemplary embodiment of the present invention, an antibacterial strength D measured by the following Method 4 is 65% or more:

[Method 4]

This method is the same as Method 1, except that 0.01 g of the antibacterial composition is added instead of 0.015 g of the antibacterial composition.

According to the exemplary embodiments, excellent antibacterial properties may be provided even when the antibacterial composition is added in an amount of 0.01 g.

According to another exemplary embodiment of the present invention, the antibacterial strength D may be 65% or more, 68% or more, 68.2% or more, and 70% or more. Further, the antibacterial strength D may be 99% or more, preferably 99.3% or more, more preferably 99.5% or more, even more preferably 99.7% or more, and still even more preferably 99.9% or more.

According to still another exemplary embodiment of the present invention, an antibacterial strength B measured by the following Method 2 is 55% or more:

[Method 2]

This method is the same as Method 1, except that 0.005 g of the antibacterial composition is added instead of 0.015 g of the antibacterial composition.

According to the exemplary embodiments, excellent antibacterial properties may be provided even when the antibacterial composition is added in an amount of 0.005 g.

According to another exemplary embodiment of the present invention, the antibacterial strength B may be 55% or more, 59% or more, 59.3% or more, 60% or more, and 61.2% or more. In addition, the antibacterial strength B may be 99% or more, preferably 99.3% or more, more preferably 99.5% or more, even more preferably 99.7% or more, and still even more preferably 99.9% or more.

According to still another exemplary embodiment of the present invention, the antibacterial composition according to the above-described exemplary embodiments has an acute oral toxicity dose LD50 of more than 300 mg/Kg, 500 mg/Kg or more, preferably 800 mg/Kg or more, and more preferably 1,000 mg/Kg or more. According to specific examples, the antibacterial composition according to the above-described exemplary embodiments may have an acute oral toxicity dose LD50 of 800 mg/Kg or more, 843 mg/Kg or more, 869 mg/Kg or more, 900 mg/Kg or more, or 998 mg/Kg or more. The antibacterial composition according to the above-described exemplary embodiments may have an acute oral toxicity dose LD50 of 1,000 mg/Kg or more, 1,050 mg/Kg or more, 1,078 mg/Kg or more, 1,100 mg/Kg or more, 1,111 mg/Kg or more, 1,200 mg/Kg or more, 1,242 mg/Kg or more, 1,300 mg/Kg or more, 1,369 mg/Kg or more, 1,400 mg/Kg or more, 1,442 mg/Kg or more, 1,498 mg/Kg or more, 1,500 mg/Kg or more, 1,600 mg/Kg or more, 1,700 mg/Kg or more, and 1,708 mg/Kg or more. According to specific examples, the antibacterial composition according to the above-described exemplary embodiments may have an acute oral toxicity dose LD50 of 843 mg/Kg, 869 mg/Kg, 998 mg/Kg, 1,078 mg/Kg, 1,111 mg/Kg, 1,242 mg/Kg, 1,369 mg/Kg, 1,442 mg/Kg, 1,498 mg/Kg, or 1,708 mg/Kg.

The higher the acute oral toxicity dose LD50 of the antibacterial composition according to the above-described exemplary embodiments, the lower the toxicity, so that the high acute oral toxicity dose is advantageous. However, the value of LD50 may be determined from the viewpoint that the above-described antibacterial strength needs also be satisfied. For example, the antibacterial composition according to the above-described exemplary embodiments may have an acute oral toxicity dose LD50 of 50,000 mg/Kg or less, for example, 10,000 mg/Kg or less, 5,000 mg/Kg or less, or 2,000 mg/Kg or less. According to an example, the antibacterial composition according to the above-described exemplary embodiments may have an acute oral toxicity dose LD50 of 1,708 mg/Kg or less.

According to an exemplary embodiment of the present invention, the compound comprising a quaternary ammonium structure having an acrylate group or methacrylate group may be selected among those capable of exhibiting the above-described antibacterial strength and acute oral toxicity dose LD50 among the compounds represented by the following Chemical Formula 1:

[Chemical Formula 1]

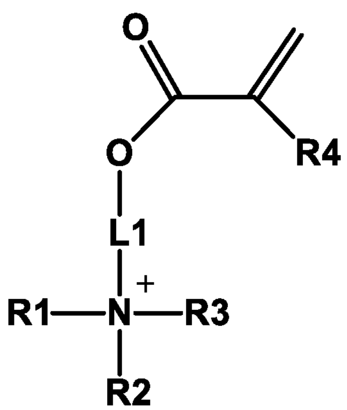

in Chemical Formula 1,

L1 is an alkylene having 2 to 4 carbon atoms,

R1, R2 and R3 are the same as or different from each other, and are each an alkyl group having 1 to 20 carbon

atoms, and
R4 is hydrogen or a methyl group.

According to an exemplary embodiment, at least one of R1, R2 and R3 of Chemical Formula 1 is an alkyl group having 8 or more carbon atoms, preferably an alkyl group having 8 to 14 carbon atoms, and more preferably an alkyl group having 8 to 12 carbon atoms.

According to an exemplary embodiment, the sum of the number of carbon atoms of the alkyl group comprised in R1, R2 and R3 of Chemical Formula 1 is 12 to 24.

According to an exemplary embodiment, the sum of the number of carbon atoms of two of R1, R2 and R3 having the larger number of carbon atoms in Chemical Formula 1 is 2 to 24, preferably 8 to 20, and more preferably 8 to 16. According to an example, the sum of the number of carbon atoms of two of R1, R2 and R3 having the larger number of carbon atoms in Chemical Formula 1 may be 9 to 15.

According to an exemplary embodiment, the number of carbon atoms of that having the largest number of carbon atoms in R1, R2 and R3 of Chemical Formula 1 is 12 or less, preferably 11 or less.

According to an exemplary embodiment, when any one of two having the largest number of carbon atoms among R1, R2 and R3 of Chemical Formula 1 has 10 or more carbon atoms, the other has less than 8 carbon atoms.

Here, when a group having the largest number of carbon atoms is selected, any one is selected in the case where there are groups having the same carbon atoms.

Acute oral toxicity may be controlled when having the number of carbon atoms according to the aforementioned exemplary embodiments. When acute oral toxicity is too high, there are restrictions on intended uses, particularly use in infant products such as diapers.

According to an exemplary embodiment of the present invention, the antibacterial composition has excellent characteristics against acute percutaneous toxicity. In general, the greater the number of carbon atoms comprised in the compound and the longer the length of the alkyl chain, the greater the toxicity that is harmful to the human body, whereas the compound comprising the quaternary ammonium structure comprised in the antibacterial composition according to the present invention exhibits reduced acute percutaneous toxicity even when the length of the alkyl chain is increased. Based on this, there is an advantage in that acute percutaneous toxicity can be controlled by adjusting the number of carbon atoms of the alkyl chain of the compound comprised in the above-described antibacterial composition. When acute percutaneous toxicity is too high, there are restrictions on intended uses, particularly use in infant products such as diapers.

According to an exemplary embodiment, L1 of Chemical Formula 1 is ethylene or butylene.

According to an exemplary embodiment, R4 of Chemical Formula 1 is hydrogen.

According to an exemplary embodiment, R4 of Chemical Formula 1 is methyl.

The antibacterial composition may be composed of only a compound comprising the quaternary ammonium structure having an acrylate group or methacrylate group, and an additive or solvent may be additionally added, if necessary.

According to another exemplary embodiment of the present invention, the compound comprising the quaternary ammonium structure having the acrylate group or methacrylate group has a water solubility of 50% or more, preferably 60% or more. The solubility may be measured at room temperature.

According to still another exemplary embodiment of the present invention, the compound comprising the quaternary ammonium structure having an acrylate group or methacrylate group has an ethanol solubility of 50% or more, preferably 60% or more. The solubility may be measured at room temperature.

According to yet another exemplary embodiment of the present invention, the compound comprising the quaternary ammonium structure having an acrylate group or methacrylate group may be dissolved in at least one of methanol, acetone, dichloromethane, DMSO, THF, and chloroform, preferably in all of them.

According to an exemplary embodiment of the present invention, a critical micelle concentration of the compound comprising the quaternary ammonium structure is 0.1 wt% to 3 wt%. In this case, the critical micelle concentration of the compound may be measured by the following Method 5.

[Method 5]

A solution comprising the compound comprising quaternary ammonium structure is prepared, and the surface tension of the solution is measured at each concentration. A calibration curve of the surface tension at each concentration is obtained, and the critical micelle concentration of the compound comprising the quaternary ammonium structure is obtained therefrom.

In this case, the surface tension at each concentration may be measured using a surface tension meter (force tensiometer) and a Du Nouy ring method.

In addition, the solution may use a solvent such as distilled water, but the solvent is not limited thereto.

According to an exemplary embodiment, the critical micelle concentration of the compound comprising the quaternary ammonium structure is 0.1 wt% to 3 wt%; 0.15 wt% to 2.5 wt%; or 0.18 wt% to 2.2 wt%. There is an advantage in that the length of the alkyl chain introduced into the compound comprising the quaternary ammonium structure can be changed by adjusting the number of carbon atoms, thereby adjusting the surface tension and solvent solubility of the compound comprising the quaternary ammonium structure.

According to yet another exemplary embodiment of the present invention, the compound comprising the quaternary ammonium structure having an acrylate group or methacrylate group may be selected among the following Monomers 1 to 10:

Monomer 1

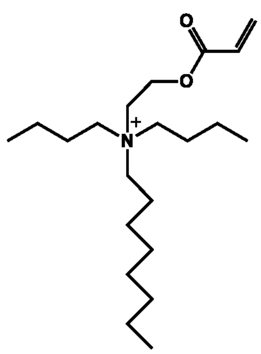

Monomer 2

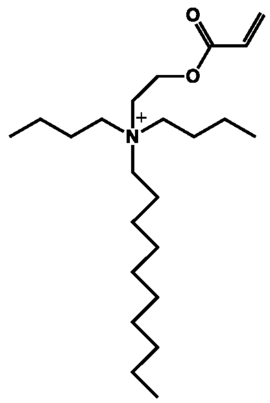

Monomer 3

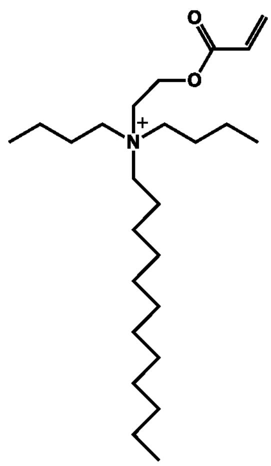

Monomer 4

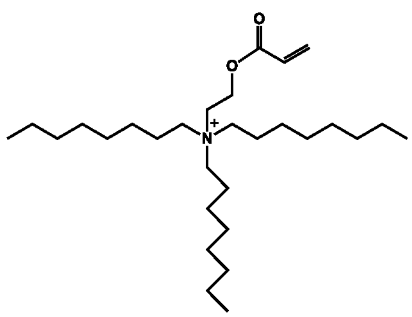

Monomer 5

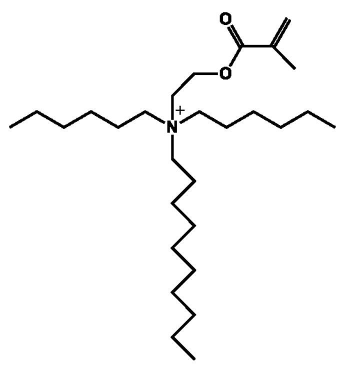

Monomer 6

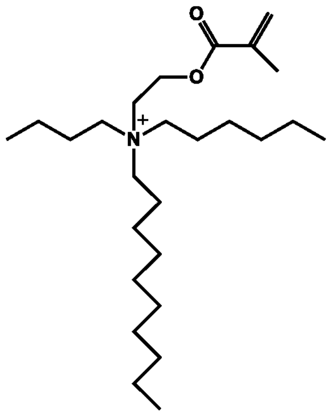

Monomer 7

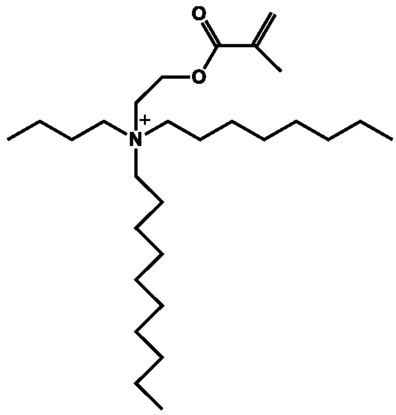

Monomer 8

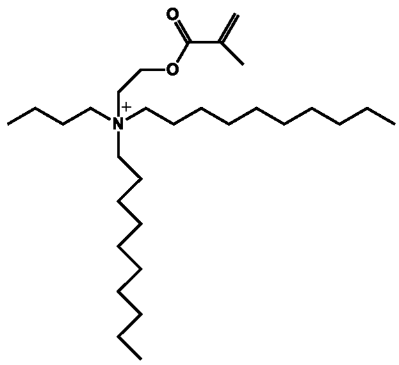

Monomer 9

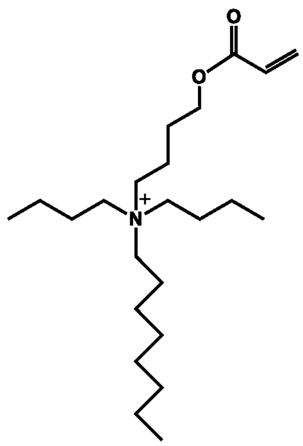

Monomer 10

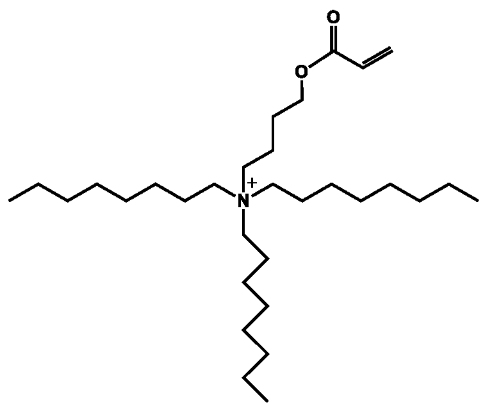

The antibacterial composition according to the above-described exemplary embodiments or the compound comprising the quaternary ammonium structure having an acrylate group or methacrylate group comprised in the same may be present in the form of a powder or oil.

Since the compound comprising the quaternary ammonium structure having an acrylate group or methacrylate group exhibits cationic properties, the compound may be present in a form where a salt is formed along with a group exhibiting anionic properties. In this case, the group exhibiting anionic properties is not particularly limited, and materials known in the art may be used as long as the materials do not impair the purpose of the antibacterial composition. For example, the group exhibiting anionic properties may be a halogen anion, specifically, $Br^-$.

According to yet another exemplary embodiment of the present invention, provided is a product comprising the antibacterial composition according to the above-described exemplary embodiments or prepared therefrom. The product is not particularly limited as long as antibacterial properties are required. According to an example, the product may be used in a state of a copolymer such as a copolymer of the antibacterial composition with a (meth)acrylate-based resin, with polyvinyl chloride, with polylactic acid (PLA), and with urethane, or may be a product comprising at least one of the copolymers, for example, a hygiene product, an antibacterial film, a diaper, and the like. The copolymer is preferably a copolymer with an acrylate or methacrylate-based compound. As the copolymerization method, a known copolymerization method may be applied.

[Mode for Invention]

Hereinafter, the present invention will be described in more detail through examples. However, the following examples are provided for illustrating the present invention, and the scope of the present invention is not limited thereby.

**[Synthesis of compound comprising quaternary ammonium structure]**

**Synthesis of Monomer 1, Monomer 2, and Monomer 3**

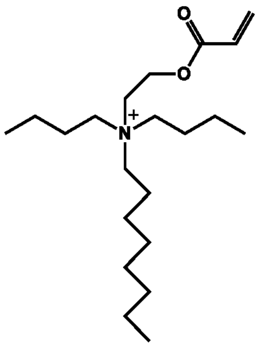

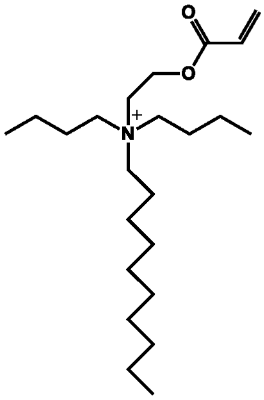

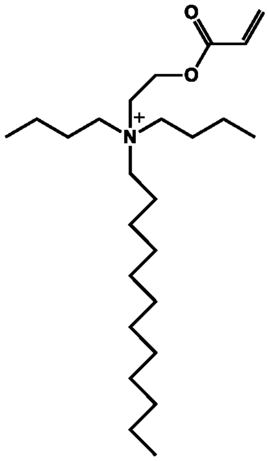

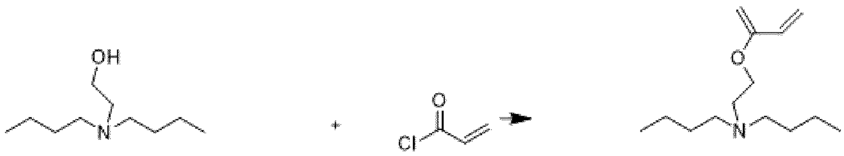

Step 1

1. 0.1 mol of 2-(dibutylamino)ethanol (DBAE), 0.1 mol of trimethylamine and 0.001 mol of hydroquinone were added to 100 mL of THF (solvent).
2. 0.1 mol of acryloyl chloride was added dropwise onto a reaction solution while stirring the materials (room temperature).
3. The resulting mixture was stirred for 2 hours.
4. After a triethylamine salt was removed by filtering the mixture, the solvent was removed by a rotary evaporator.
5. The residue was dried under vacuum at 83 to 87°C.

Step 2

1. The product of Step 1 and 1-bromooctane (preparation of Monomer 1), 1-bromodecane (preparation of Monomer 2), or 1-bromododecane (preparation of Monomer 3) were dissolved at 50 wt% in acrylonitrile (solvent) at a ratio of 1:1.
2. Subsequently, p-methoxyphenol, which is a polymerization inhibitor, was added (ratio with reactants 1:0.001 eq).
3. The resulting mixture was reacted at 50°C for 20 hours.
4. After static precipitation (MTBE:reaction solution = 15:1) in methyl t-butyl ether (MTBE), the mixture was filtered. Here, although a static precipitation method of adding a reactant to a nonsolvent was used, a reverse precipitation method of adding a nonsolvent to a reactant may also be used. Furthermore, in addition to 15:1, other ratios of MTBE and reaction solution may be used, and for example, 12:1 and 26:1 may be used.
5. The resulting product was dried under vacuum at 45°C.

The $^1$H-NMR spectrum of synthesized Monomer 1 is illustrated in the following FIG. 1, the $^1$H-NMR spectrum of synthesized Monomer 2 is illustrated in the following FIG. 2, and the $^1$H-NMR spectrum of synthesized Monomer 3 is illustrated in the following FIG. 3.

**Synthesis of Monomer 4**

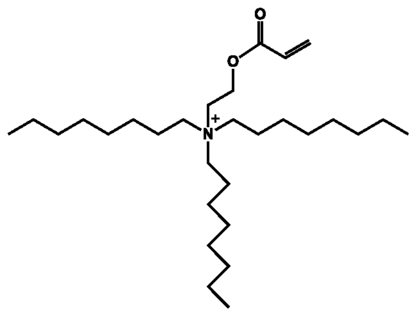

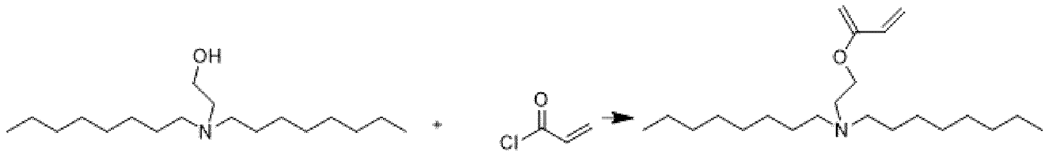

Step 1

Monomer 4 was prepared in the same manner as in Step 1 of Synthesis of Monomer 1, Monomer 2, and Monomer 3, except that 2-(dioctylamino)ethanol was used instead of 2-(dibutylamino)ethanol.

Step 2

Monomer 4 was prepared in the same manner as in Step 2 of Monomer 1 of Synthesis of Monomer 1, Monomer 2, and Monomer 3.

Synthesis of Monomer 4 was confirmed by $^1$H-NMR spectrum ($(CD_3)_2SO$) in a manner similar to that of the above-described Monomers 1 to 3.

**Synthesis of Monomer 5**

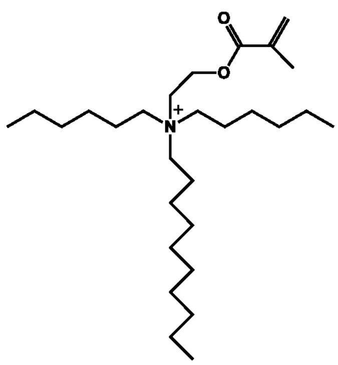

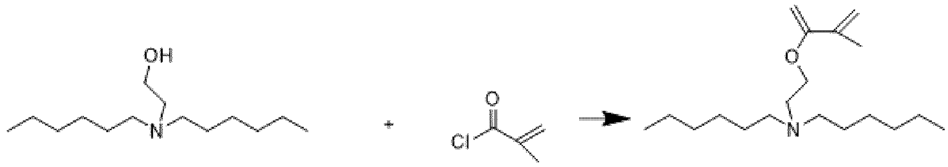

Step 1

Monomer 5 was prepared in the same manner as in Step 1 of Synthesis of Monomer 1, Monomer 2, and Monomer 3, except that 2-(dihexylamino)ethanol was used instead of 2-(dibutylamino)ethanol.

Step 2

Monomer 5 was prepared in the same manner as in Step 2 of Monomer 2 of Synthesis of Monomer 1, Monomer 2, and Monomer 3.

Synthesis of Monomer 5 was confirmed by $^1$H-NMR spectrum ($(CD_3)_2SO$) in a manner similar to that of the above-described Monomers 1 to 3.

**Synthesis of Monomer 6**

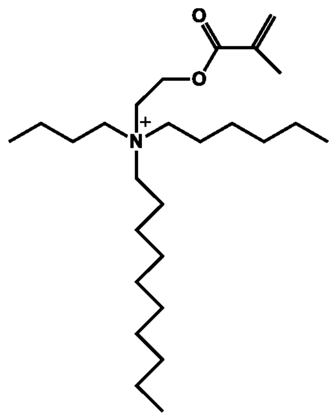

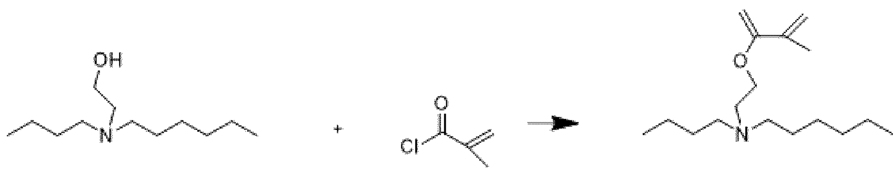

Step 1

Monomer 6 was prepared in the same manner as in Step 1 of Synthesis of Monomer 1, Monomer 2, and Monomer 3, except that 2-(butylhexylamino)ethanol and methacryloyl chloride were used instead of 2-(dibutylamino)ethanol and acryloyl chloride, respectively.

Step 2

Monomer 6 was prepared in the same manner as in Step 2 of Monomer 2 of Synthesis of Monomer 1, Monomer 2, and Monomer 3.

Synthesis of Monomer 6 was confirmed by $^1$H-NMR spectrum ($(CD_3)_2SO$) in a manner similar to that of the above-described Monomer 1 to 3.

**Synthesis of Monomer 7**

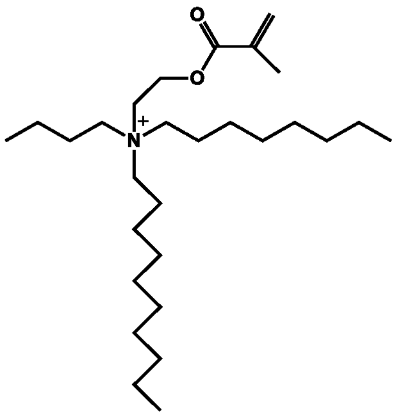

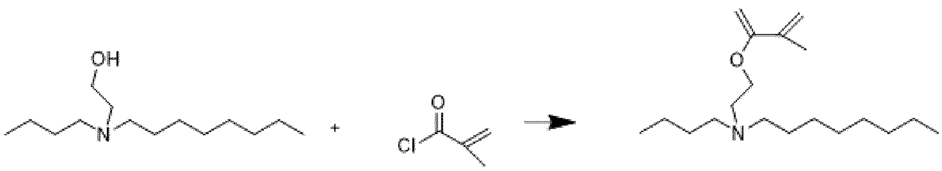

Step 1

Monomer 7 was prepared in the same manner as in Step 1 of Synthesis of Monomer 1, Monomer 2, and Monomer 3, except that 2-(butyloctylamino)ethanol and methacryloyl chloride were used instead of 2-(dibutylamino)ethanol and acryloyl chloride, respectively.

Step 2

Monomer 7 was prepared in the same manner as in Step 2 of Monomer 2 of Synthesis of Monomer 1, Monomer 2, and Monomer 3.

Synthesis of Monomer 7 was confirmed by $^1$H-NMR spectrum ($(CD_3)_2SO$) in a manner similar to that of the above-described Monomer 1 to 3.

**Synthesis of Monomer 8**

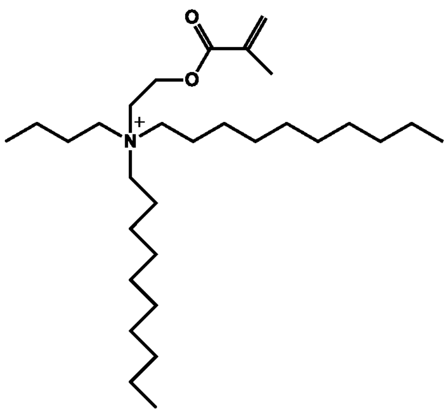

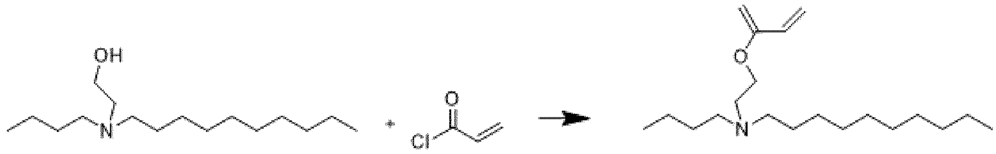

Step 1

Monomer 8 was prepared in the same manner as in Step 1 of Synthesis of Monomer 1, Monomer 2, and Monomer 3, except that 2-(butyldecylamino)ethanol and methacryloyl chloride were used instead of 2-(dibutylamino)ethanol and acryloyl chloride, respectively.

Step 2

Monomer 8 was prepared in the same manner as in Step 2 of Monomer 2 of Synthesis of Monomer 1, Monomer 2, and Monomer 3.

Synthesis of Monomer 8 was confirmed by $^1$H-NMR spectrum ($(CD_3)_2SO$) in a manner similar to that of the above-described Monomer 1 to 3.

**Synthesis of Monomer 9**

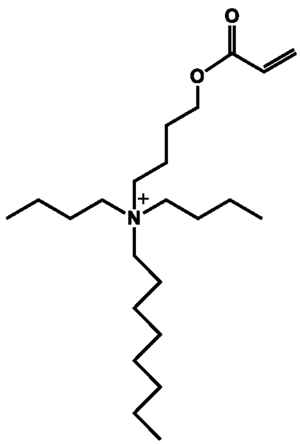

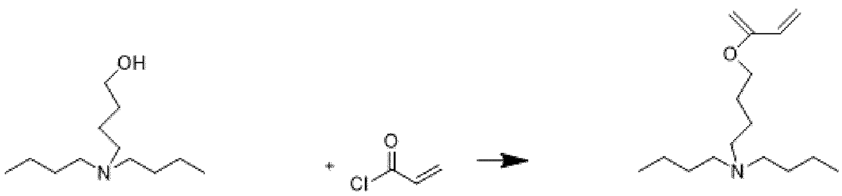

Step 1

Monomer 9 was prepared in the same manner as in Step 1 of Synthesis of Monomer 1, Monomer 2, and Monomer 3, except that 2-(dibutylamino)butanol was used instead of 2-(dibutylamino)ethanol.

Step 2

Monomer 9 was prepared in the same manner as in Step 2 of Monomer 1 of Synthesis of Monomer 1, Monomer 2, and Monomer 3.

Synthesis of Monomer 9 was confirmed by $^1$H-NMR spectrum ($(CD_3)_2SO$) in a manner similar to that of the above-described Monomers 1 to 3.

**Synthesis of Monomer 10**

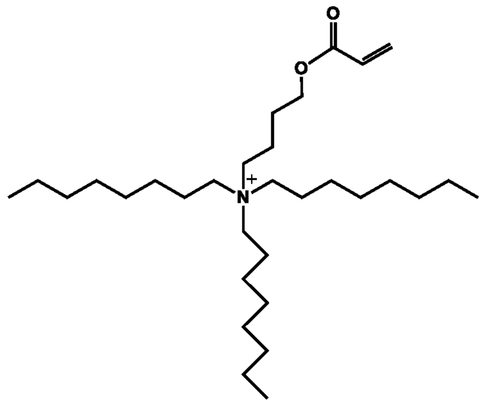

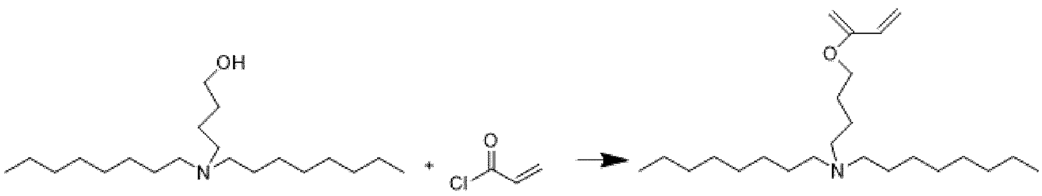

Step 1

Monomer 10 was prepared in the same manner as in Step 1 of Synthesis of Monomer 1, Monomer 2, and Monomer 3, except that 2-(dioctylamino)butanol was used instead of 2-(dibutylamino)ethanol.

Step 2

Monomer 10 was prepared in the same manner as in Step 2 of Monomer 1 of Synthesis of Monomer 1, Monomer 2, and Monomer 3.

Synthesis of Monomer 10 was confirmed by $^1$H-NMR spectrum ($(CD_3)_2SO$) in a manner similar to that of the above-described Monomer 1 to 3.

**Comparative Monomer 1**

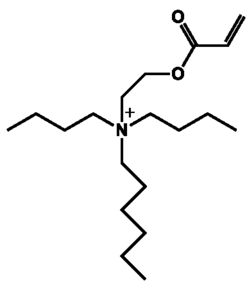

Synthesis was performed in the same manner as in the synthesis process of Monomer 1, except that bromohexane was used instead of 1-bromooctane of Step 2 in the synthesis process of Monomer 1.

Synthesis of Comparative Monomer 1 was confirmed by $^1$H-NMR spectrum ($(CD_3)_2SO$) in a manner similar to that of the above-described Monomers 1 to 3.

**Comparative Monomer 2**

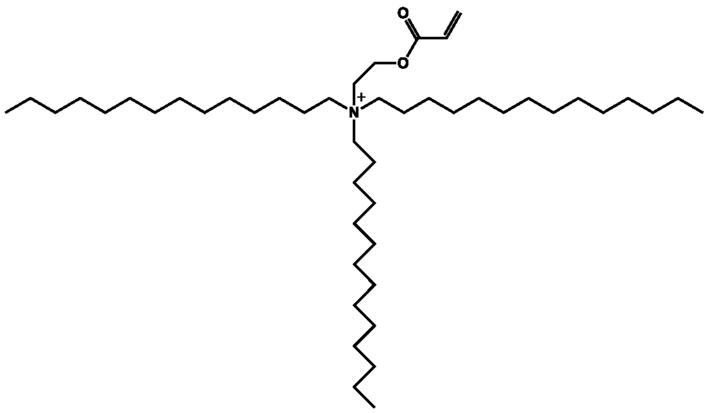

Synthesis was performed in the same manner as in the synthesis process of Monomer 10, except that 2-(ditetradecylamino)butanol was used instead of 2-(dioctylamino)butanol of Step 1 and bromotetradecene was used instead of 1-bromooctane of Step 2 in the synthesis process of Monomer 10.

Synthesis of Comparative Monomer 2 was confirmed by $^{1}$H-NMR spectrum ($(CD_3)_2SO$) in a manner similar to that of the above-described Monomers 1 to 3.

The antibacterial strength of Monomers 1 to 10 and Comparative Monomers 1 and 2 by Methods 1 to 4 was measured, and is shown in the following Table 1. In this case, the *P.mirabilis* (ATCC29906) bacteria were used.

[Table 1]

| No. | Sample | Antibacterial strength (%) | | | |
|---|---|---|---|---|---|
| | | Method 2 (amount of antibacterial composition added 0.005g) | Method 4 (amount of antibacterial composition added 0.01g) | Method 1 (amount of antibacterial composition added 0.015g) | Method 3 (amount of antibacterial composition added 0.02g) |
| 1 | reference | - | - | - | - |
| 2 | Monomer 1 | 59.3 | 68.2 | 99.9 | 99.9 |
| 3 | Monomer 2 | 99.9 | 99.9 | 99.9 | 99.9 |
| 4 | Monomer 3 | 99.9 | 99.9 | 99.9 | 99..9 |
| 5 | Monomer 4 | 99.9 | 99.9 | 99.9 | 99.9 |
| 6 | Monomer 5 | 99.9 | 99.9 | 99.9 | 99.9 |
| 7 | Monomer 6 | 99.9 | 99.9 | 99.9 | 99.9 |
| 8 | Monomer 7 | 99.9 | 99.9 | 99.9 | 99.9 |
| 9 | Monomer 8 | 99.9 | 99.9 | 99.9 | 99.9 |
| 10 | Monomer 9 | 61.2 | 70.0 | 99.9 | 99.9 |
| 11 | Monomer 10 | 99.9 | 99.9 | 99.9 | 99.9 |
| 12 | Comparative Monomer 1 | 22.6 | 30.7 | 38.8 | 46.9 |
| 13 | Comparative Monomer 2 | 99.9 | 99.9 | 99.9 | 99.9 |

According to Table 1, all Monomers 1 to 10 had an antibacterial strength A of 99.9% measured by Method 1.

The time at which 99.9% of the antibacterial strength of Monomers 1 to 10 and Comparative Monomers 1 and 2 is confirmed is shown in the following Table 2.

[Table 2]

| No. | Sample | Antibacterial strength (%) | | | |
|---|---|---|---|---|---|
| | | Method 2 (amount of antibacterial composition added 0.005g) | Method 4 (amount of antibacterial composition added 0.01g) | Method 1 (amount of antibacterial composition added 0.015g) | Method 3 (amount of antibacterial composition added 0.02g) |
| 1 | reference | - | - | - | - |
| 2 | Monomer 1 | - | - | Within 1 hour | Within 1 hour |
| 3 | Monomer 2 | Within 1 hour | Within 1 hour | Within 1 hour | Within 1 hour |
| 4 | Monomer 3 | Within 1 hour | Within 1 hour | Within 1 hour | Within 1 hour |
| 5 | Monomer 4 | Within 1 hour | Within 1 hour | Within 1 hour | Within 1 hour |
| 6 | Monomer 5 | Within 1 hour | Within 1 hour | Within 1 hour | Within 1 hour |
| 7 | Monomer 6 | Within 1 hour | Within 1 hour | Within 1 hour | Within 1 hour |
| 8 | Monomer 7 | Within 1 hour | Within 1 hour | Within 1 hour | Within 1 hour |
| 9 | Monomer 8 | Within 1 hour | Within 1 hour | Within 1 hour | Within 1 hour |
| 10 | Monomer 9 | - | - | Within 1 hour | Within 1 hour |
| 11 | Monomer 10 | Within 1 hour | Within 1 hour | Within 1 hour | Within 1 hour |
| 12 | Comparative Monomer 1 | - | - | - | - |
| 13 | Comparative Monomer 2 | Within 1 hour | Within 1 hour | Within 1 hour | Within 1 hour |

According to Table 2, all Monomers 1 to 10 having an antibacterial strength A of 99.9% measured by Method 1 was confirmed within 1 hour.

The acute oral toxicity dose LD50 of Monomers 1 to 10 and Comparative Monomers 1 and 2 is shown in the following Table 3.

[Table 3]

| Sample | Acute oral toxicity dose (mg/Kg) LD50 |
|---|---|
| Monomer 1 | 1,708 |
| Monomer 2 | 1,369 |
| Monomer 3 | 843 |
| Monomer 4 | 1,442 |
| Monomer 5 | 1,078 |
| Monomer 6 | 1,111 |
| Monomer 7 | 998 |
| Monomer 8 | 869 |
| Monomer 9 | 1,498 |
| Monomer 10 | 1,242 |
| Comparative Monomer 1 | 2,355 |
| Comparative Monomer 2 | 231 |

According to Table 3, all Monomers 1 to 10 had an acute oral toxicity dose LD50 of 300 mg/Kg or more, particularly 800 mg/Kg or more. Furthermore, Examples 1, 2, 4, 5, 6, 9 and 10 had an acute oral toxicity dose LD50 of 1,000 mg/Kg or more. The acute oral toxicity dose LD50 was measured by 3T3 Neutral Red Uptake (NRU) assay (OECD Guidance Document No 129). Specifically, LD50 may be calculated by the following method.

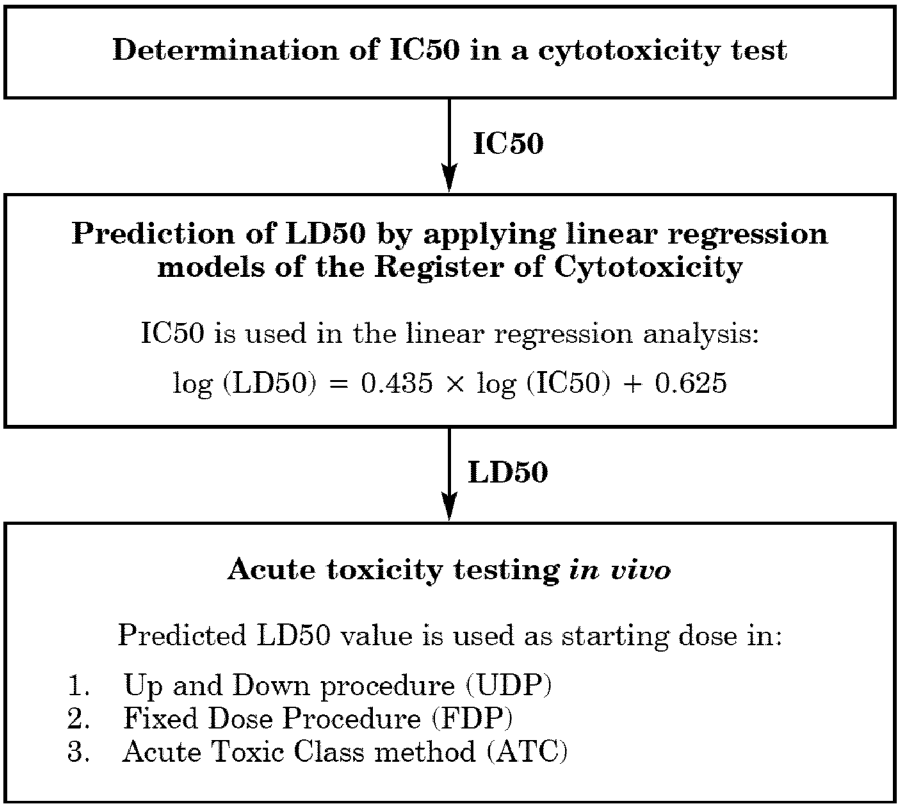


The critical micelle concentrations of Monomers 2 and 3 and Comparative Monomer 2 were measured by Method 5, and are shown in the following Table 4 and FIGS. 4 to 6. More specifically, aqueous solutions (30 mL) were prepared for each concentration of Monomers 2 and 3 and Comparative Monomer 2, and the surface tension was measured using the Du Nouyring method of a force tensiometer. Each measurement was repeated three times. The surface tension was measured by preparing diluted solutions until the surface tension was similar to deionized water. The critical micelle concentration (CMC) was confirmed by confirming through the change in surface tension with concentration that each aqueous solution formed a micelle.

FIG. 4 is a view illustrating the surface tension of Monomer 2 at each concentration, FIG. 5 is a view illustrating the surface tension of Monomer 3 at each concentration, and FIG. 6 is a view illustrating the surface tension of Comparative Monomer 2 at each concentration.

[Table 4]

| Sample | Critical micelle concentration (wt%) |
|---|---|
| reference (Deionized water) | - |
| Monomer2 | 1.5 ± 0.3 |
| Monomer3 | 0.25 ± 0.05 |
| Comparative Monomer2 | 0.06 ± 0.01 |

According to Table 4, it can be confirmed that the critical micelle concentration changes according to the length of the alkyl chain in the monomer, and it can be confirmed that the longer the alkyl chain, the lower the critical micelle concentration.

Therefore, according to an exemplary embodiment of the present invention, the physical properties of the antibacterial composition can be adjusted such that an antibacterial effect and water solubility can be adjusted by

adjusting the number of carbon atoms of the compound comprising a quaternary ammonium structure having an acrylate group or a methacrylate group.

## Claims

1. An antibacterial composition comprising one or more compounds comprising a quaternary ammonium structure having an acrylate group or a methacrylate group,
wherein a critical micelle concentration of the compound comprising the quaternary ammonium structure is 0.1 wt% to 3 wt%.

2. The antibacterial composition of claim 1, wherein the critical micelle concentration of the compound comprising the quaternary ammonium structure is 0.18 wt% to 2.2 wt%.

3. The antibacterial composition of claim 1, wherein the antibacterial composition has an antibacterial strength A of 90% or more which are measured by the following Method 1 for at least one strain of Gram-positive bacteria and Gram-negative bacteria and an acute oral toxicity dose LD50 of 300 mg/Kg or more:
[Method 1]

After 25 ml of a broth-type medium (Nutrient broth, BD DIFCO., 8 g/L) inoculated with 3,000 CFU/ml bacteria is put into a 50 mL conical tube, 0.015 g of the antibacterial composition is added thereto and suspended (vortexing), a sufficiently mixed solution is incubated for 16 hours in a shaking water bath maintained at 35°C. After the incubated solution is diluted to 1/5 using a 1X PBS buffer solution, absorbance ($\lambda$ = 600 nm) is measured using a UV/Vis spectrophotometer, and the antibacterial strength, which is a bacteriostatic reduction rate, is calculated by the following equation by comparing the measured absorbance with a solution incubated without the addition of the antibacterial composition.

$$\text{Antibacterial strength (\%)} = (1 - A_{sample}/A_{Reference}) \times 100$$

$$A_{sample} = \text{Absorbance of medium solution incubated by adding antibacterial composition}$$

$$A_{Reference} = \text{Absorbance of medium solution incubated without addition of antibacterial composition}$$

4. The antibacterial composition of claim 3, wherein Method 1 is measured against at least one strain of *Proteus mirabilis, E.coli, S. aureus, E.cloacae* and *E.faecalis*.

5. The antibacterial composition of claim 3, wherein Method 1 is measured for each strain of *Proteus mirabilis, E.coli, S.aureus, E.cloacae* and *E.faecalis*.

6. The antibacterial composition of claim 3, wherein an antibacterial strength A of the antibacterial composition is expressed within 1 hour.

7. The antibacterial composition of claim 3, wherein a ratio (CB) of an antibacterial strength C measured by the following Method 3 to an antibacterial strength B measured by the following Method 2 is 1 or more and less than 2:

[Method 2]

This method is the same as Method 1, except that 0.005 g of the antibacterial composition is added instead of 0.015 g of the antibacterial composition.
[Method 3]
This method is the same as Method 1, except that 0.02 g of the antibacterial composition is added instead of 0.015 g of the antibacterial composition.

**8.** The antibacterial composition of claim 3, wherein the antibacterial strength A is 99% or more.

**9.** The antibacterial composition of claim 3, wherein an antibacterial strength D measured by the following Method 4 is 65% or more:
[Method 4]
This method is the same as Method 1, except that 0.01 g of the antibacterial composition is added instead of 0.015 g of the antibacterial composition.

**10.** The antibacterial composition of claim 9, wherein the antibacterial strength D is 99% or more.

**11.** The antibacterial composition of claim 3, wherein the antibacterial strength B measured by the following Method 2 is 55% or more:
[Method 2]
This method is the same as Method 1, except that 0.005 g of the antibacterial composition is added instead of 0.015 g of the antibacterial composition.

**12.** The antibacterial composition of claim 11, wherein the antibacterial strength B is 99% or more.

**13.** The antibacterial composition of claim 3, wherein the acute oral toxicity dose LD50 is 800 mg/Kg or more.

**14.** The antibacterial composition of claim 3, wherein the acute oral toxicity dose LD50 is 1,000 mg/Kg or more.

**15.** The antibacterial composition of claim 1, wherein the compound comprising the quaternary ammonium structure having an acrylate group or methacrylate group is selected among compounds represented by the following Chemical Formula 1:

[Chemical Formula 1]

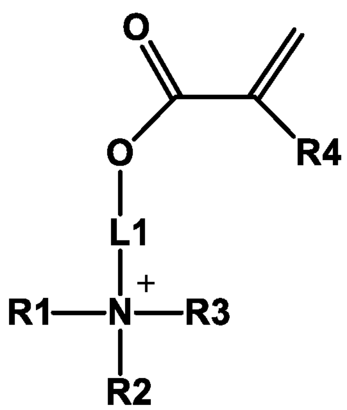

in Chemical Formula 1,

L1 is an alkylene group having 2 to 4 carbon atoms,
R1, R2 and R3 are the same as or different from each other, and are each an alkyl group having 1 to 20 carbon atoms, and
R4 is hydrogen or a methyl group.

**16.** The antibacterial composition of claim 15, wherein at least one of R1, R2 and R3 of Chemical Formula 1 is an alkyl group having 8 or more carbon atoms, and the sum of the number of carbon atoms of the alkyl group comprised in R1, R2 and R3 is 12 to 24.

**17.** The antibacterial composition of claim 1, wherein the compound comprising the quaternary ammonium structure having an acrylate group or methacrylate group is selected among the following Monomers 1 to 10:

Monomer 1

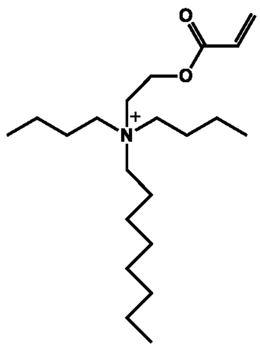

Monomer 2

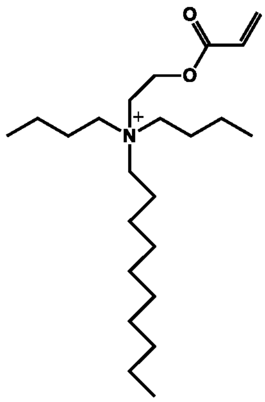

Monomer 3

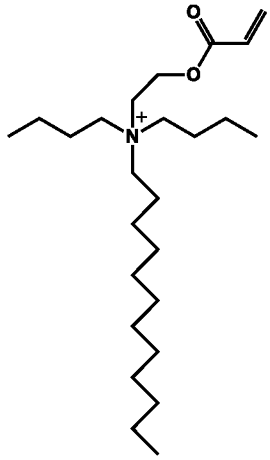

Monomer 4

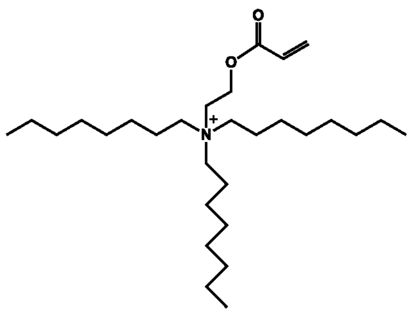

Monomer 5

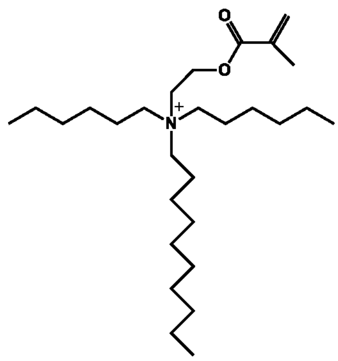

Monomer 6

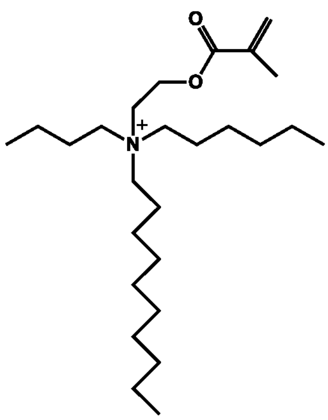

Monomer 7

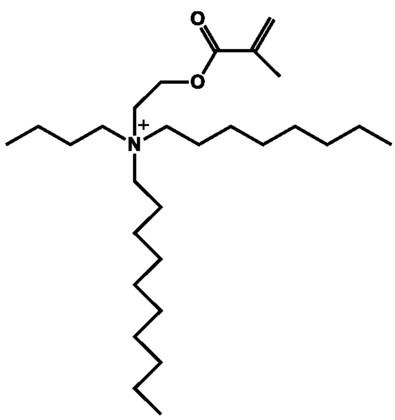

Monomer 8

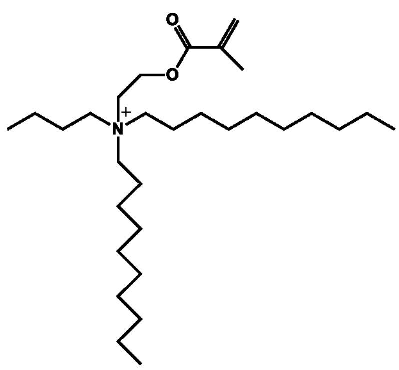

Monomer 9

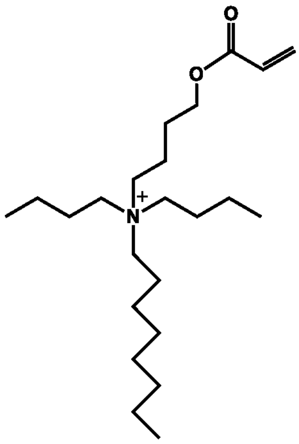

Monomer 10

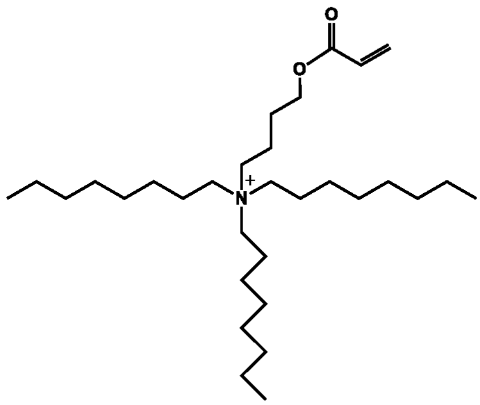

**18.** A product comprising the antibacterial composition of any one of claims 1 to 17, or prepared therefrom.

[Figure 1]

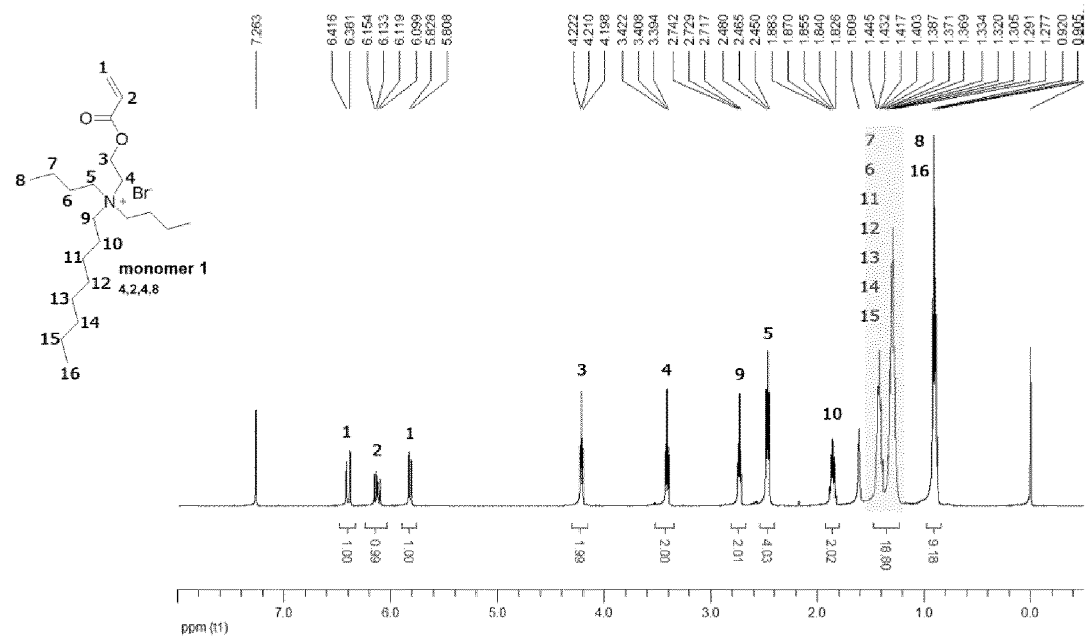


[Figure 2]

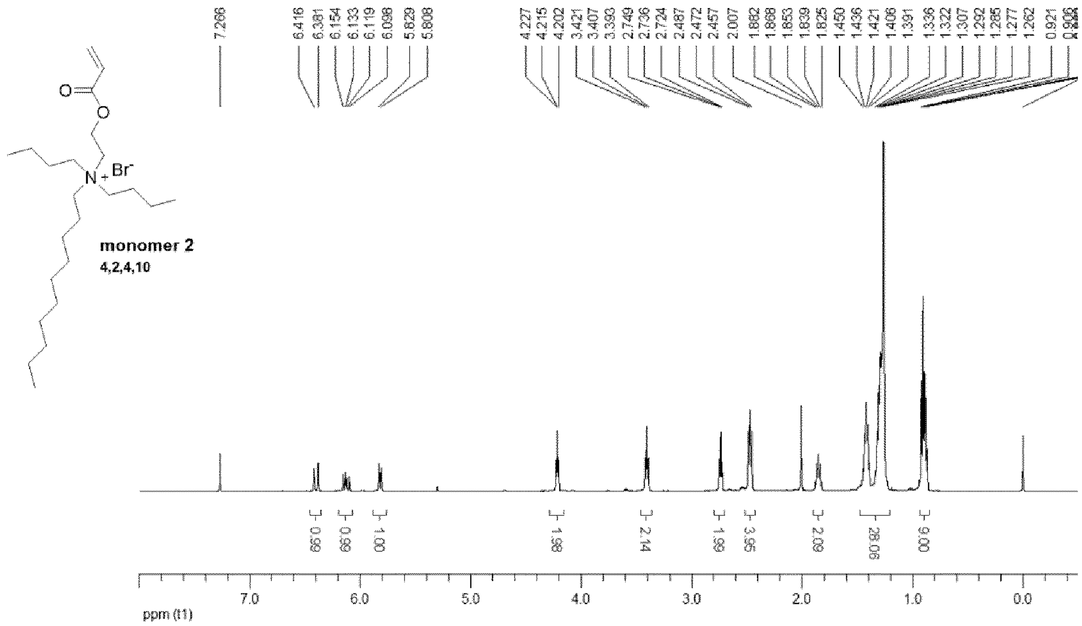

[Figure 3]

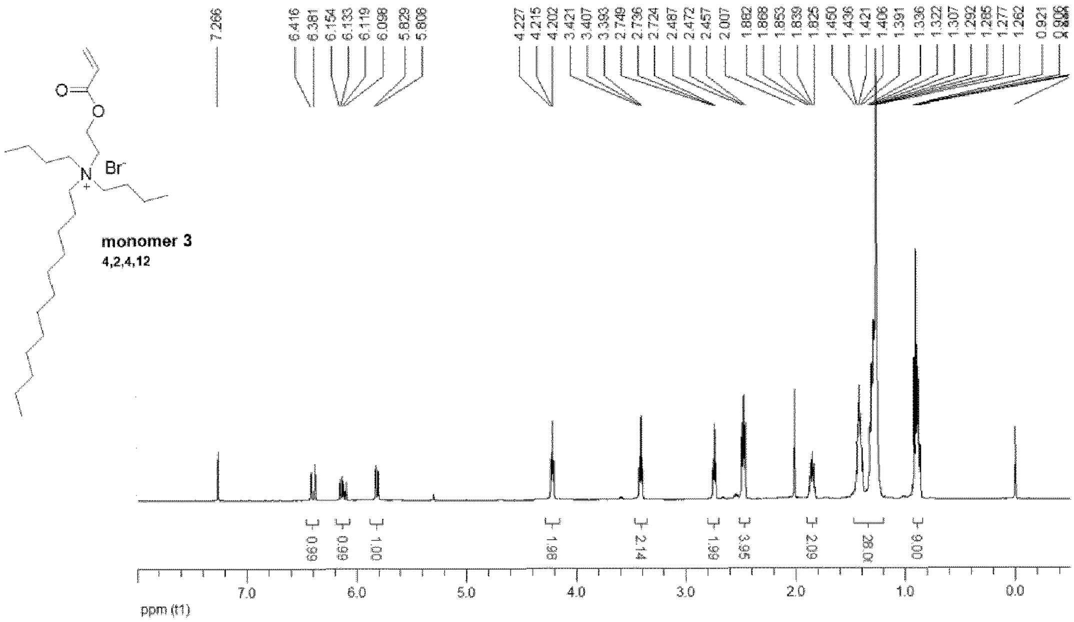


[Figure 4]

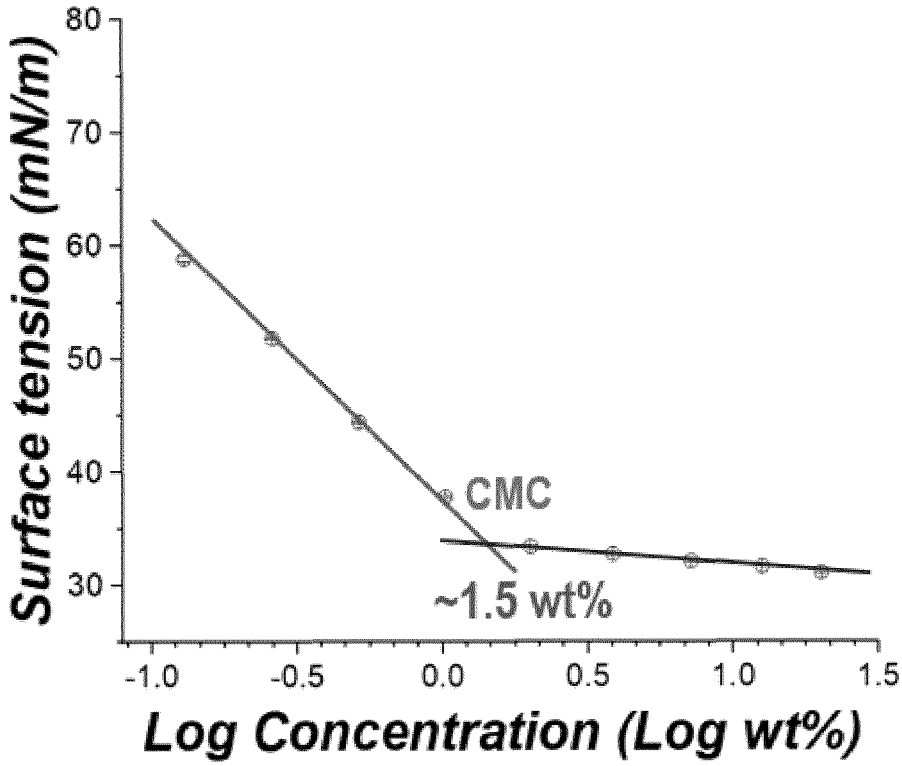

[Figure 5]

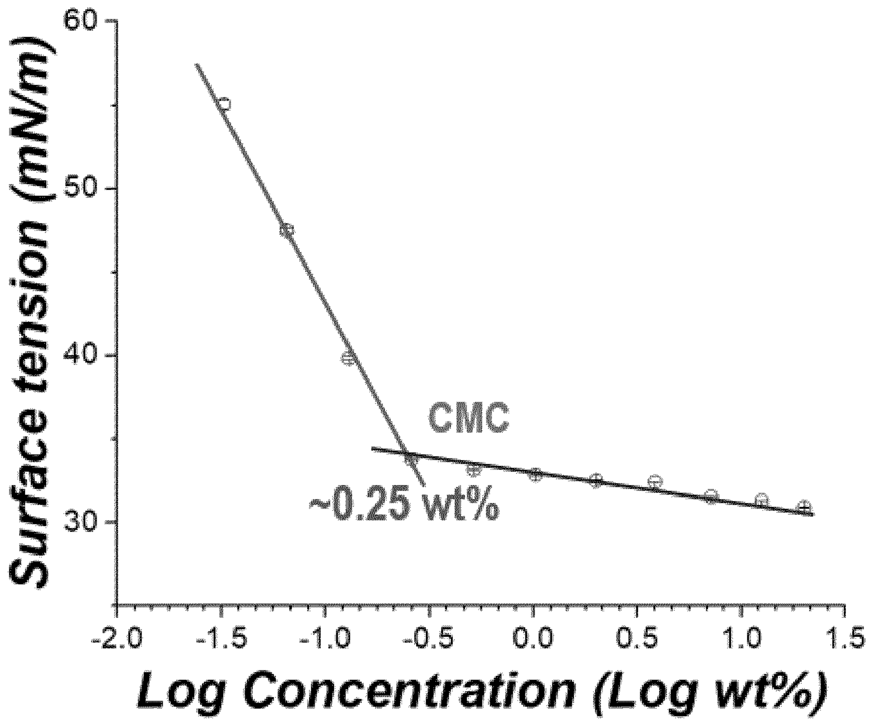


[Figure 6]

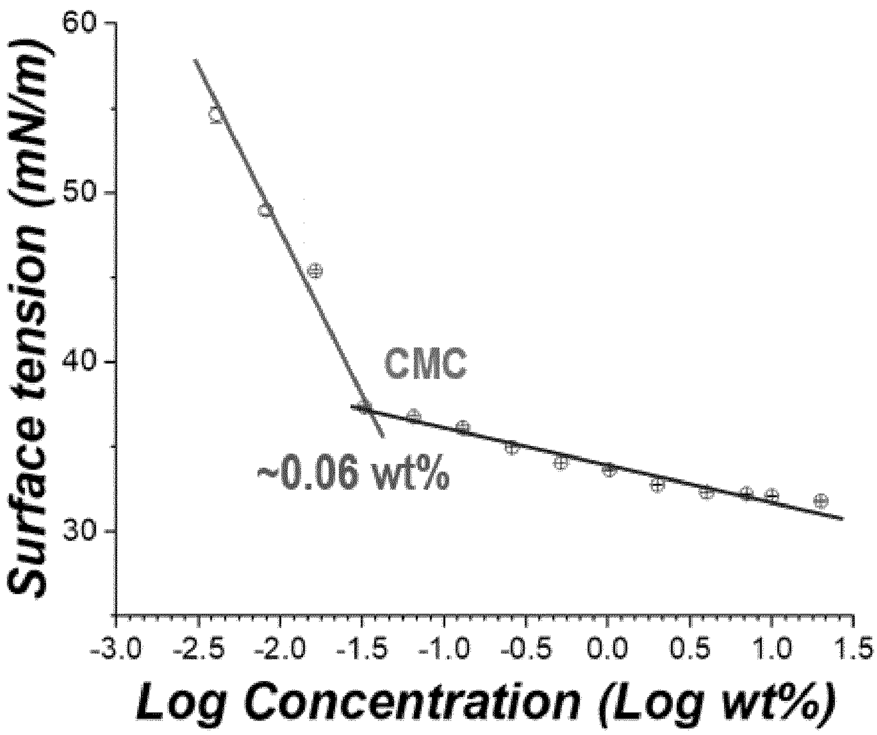

## INTERNATIONAL SEARCH REPORT

International application No.
**PCT/KR2022/010196**

### A. CLASSIFICATION OF SUBJECT MATTER

**A01N 33/12**(2006.01)i

According to International Patent Classification (IPC) or to both national classification and IPC

### B. FIELDS SEARCHED

Minimum documentation searched (classification system followed by classification symbols)

A01N 33/12(2006.01); A01N 25/04(2006.01); G02B 1/10(2006.01); G02B 5/26(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus), Google & keywords: 항균(antibacterial), 친수성(hydrophilic), 소수성(hydrophobic), 아크릴레이트(acrylate), 메타크릴레이트(methacrylate), 4급 암모늄(quaternary ammonium), 그람 양성균(gram-positive bacteria), 그람 음성균(gram-negative bacteria)

### C. DOCUMENTS CONSIDERED TO BE RELEVANT

| Category* | Citation of document, with indication, where appropriate, of the relevant passages | Relevant to claim No. |
|---|---|---|
| X | MOHAMMED, M. et al. Antibacterial activity of quaternary ammonium salt from diethylaminoethyl methacrylate. E-Journal of Chemistry. 2010, vol. 7, no. S1, pp. S61-S66.<br>See abstract; pages S62 and S65; formulas 1-3; and table 1. | 1-18 |
| X | DAOUDI, S. et al. Synthesis, Characterizationand Antibacterial Activity of Quaternary Ammonium Compounds Bearing Mixed N-Substituted Groups. Chem. Sci. Trans. 2014, vol. 3, no. 1, pp. 281-291.<br>See abstract; pages 284 and 289-290; figure 1; and table 5. | 1-18 |
| X | MANCUSO, R. et al. Synthesis and antibacterial activity of polymerizable acryloyloxyalkyltriethyl ammonium salts. ChemPlusChem. 2017, vol. 82, no. 10, pp. 1235-1244.<br>See abstract; Introduction and Antimicrobial tests; formulas 1 and 2; and page 1236, right column, lines 8-13. | 1-18 |
| A | JP 2012-208169 A (KONICA MINOLTA HOLDINGS INC.) 25 October 2012 (2012-10-25)<br>See entire document. | 1-18 |
| A | JP 2000-154105 A (DAICEL CHEM. IND. LTD.) 06 June 2000 (2000-06-06)<br>See entire document. | 1-18 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 October 2022** | **28 October 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2022/010196**

| Patent document cited in search report | Publication date (day/month/year) | Patent family member(s) | Publication date (day/month/year) |
|---|---|---|---|
| JP 2012-208169 A | 25 October 2012 | JP 5699738 B2 | 15 April 2015 |
| JP 2000-154105 A | 06 June 2000 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020210093569